# EUROPEAN PATENT APPLICATION

(11) **EP 4 740 834 A1**
(43) Date of publication of application: **13.05.2026**
(21) Application number: 25214334.2
(22) Date of filing: 07.11.2025
(51) Int. Cl.: A61B 3/036, A61B 3/032

(54) **OPHTHALMOLOGIC APPARATUS AND METHOD FOR CONTROLLING THE SAME**

(30) Priority: 11.11.2024 JP 2024196950
(71) Applicant: Topcon Corporation, Tokyo 174-8580 (JP)
(72) Inventor: SAKURADA, Tomohiro, Tokyo, 174-8580 (JP)
(74) Representative: Wagner, Jürgen

(57) **Abstract**

An ophthalmologic apparatus (1, 1A) includes a display portion (22) that is configured to display an astigmatism test chart (45, 45A) for obtaining an astigmatism axis angle of an examinee (S); and a control portion (34, 80) that is configured to control the display portion (22) to display and rotate the astigmatism test chart (45, 45A) on the display portion (22). The astigmatism test chart (45, 45A) includes a first visual target (46) including a plurality of first straight lines (46a) that are arranged in parallel with each other at a predetermined interval, and a second visual target (47) including a plurality of second straight lines (47a) that are arranged in parallel with each other at a predetermined interval and extend in a direction orthogonal to a direction of the first straight lines (46a). The first visual target (46) is displayed in a circular first area (48), and the second visual target (47) is displayed in an annular second area (49) surrounding the first area (48).

## Description

### FIELD OF THE INVENTION

The present invention relates to an ophthalmologic apparatus and a method for controlling the same.

### BACKGROUND

Prior art such as Japanese Utility Model Application Publications S52-042595 and S59-071601 discloses an ophthalmologic apparatus configured to display and rotate an astigmatism test chart including two groups of straight lines oriented at 90° to each other, enabling subjective determination of the astigmatic axis angle of an examinee. The astigmatic axis angle is determined from the examinee's simultaneous visual comparison of the two groups.

### SUMMARY

The ophthalmologic apparatus disclosed in Japanese Utility Model Application Publication S52-042595 displays an astigmatism chart in which two groups of straight lines are displayed apart from one another, and each group is enclosed by an outline that terminates the line ends at the boundary. This configuration makes simultaneous visual comparison difficult because the examinee must shift the line of sight to observe the two groups of lines.

The ophthalmologic apparatus disclosed in Japanese Utility Model Application Publication S59-071601 displays an astigmatism chart in which the opposing ends of two groups of straight lines are aligned on a common virtual straight line, either placed adjacent along the line or overlapping one another on the line. This arrangement reduces the need for the examinee to shift the line of sight during simultaneous comparison of the two groups. However, since the two groups face each other across the virtual line, further improvement in facilitating simultaneous comparison remains desirable.

The present invention has been made in view of the foregoing problem. An object of the present invention is to provide an ophthalmologic apparatus and a method for controlling the ophthalmologic apparatus that facilitate simultaneous visual comparison of two groups of straight lines extending in mutually perpendicular directions.

The present invention provides an ophthalmologic apparatus including a display portion that is configured to display an astigmatism test chart for obtaining an astigmatism axis angle of an examinee; and a control portion that is configured to control the display portion to display and rotate the astigmatism test chart on the display portion. The astigmatism test chart includes a first visual target including a plurality of first straight lines that are arranged in parallel with each other at a predetermined interval, and a second visual target including a plurality of second straight lines that are arranged in parallel with each other at a predetermined interval and extend in a direction orthogonal to a direction of the first straight lines. The first visual target is displayed in a circular first area, and the second visual target is displayed in an annular second area surrounding the first area.

The present invention also provides a method for controlling an ophthalmologic apparatus. The ophthalmologic apparatus includes a display portion that is configured to display an astigmatism test chart for obtaining an astigmatism axis angle of an examinee; and a control portion that is configured to control the display portion to display and rotate the astigmatism test chart on the display portion. The method includes displaying the astigmatism test chart on the display portion. The astigmatism test chart includes a first visual target including a plurality of first straight lines that are arranged in parallel with each other at a predetermined interval, and a second visual target including a plurality of second straight lines that are arranged in parallel with each other at a predetermined interval and extend in a direction orthogonal to a direction of the first straight lines. The displaying the astigmatism test chart includes displaying the first visual target in a circular first area and displaying the second visual target in an annular second area surrounding the first area.

According to the ophthalmologic apparatus and the method for controlling the ophthalmologic apparatus of the present invention with the above configuration, the simultaneous visual comparison of two groups of straight lines extending in mutually perpendicular directions is facilitated.

### BRIEF DESCRIPTION OF DRAWINGS

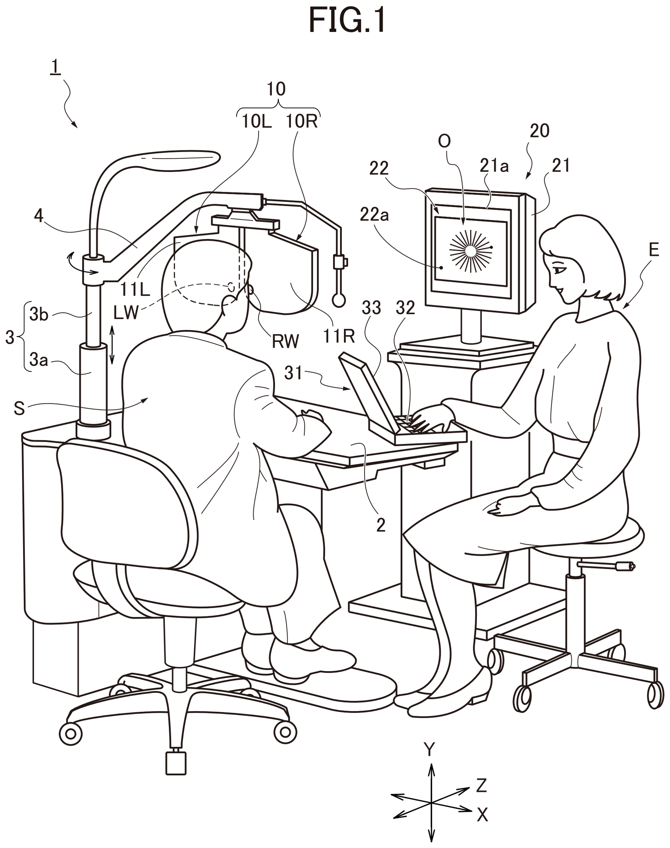

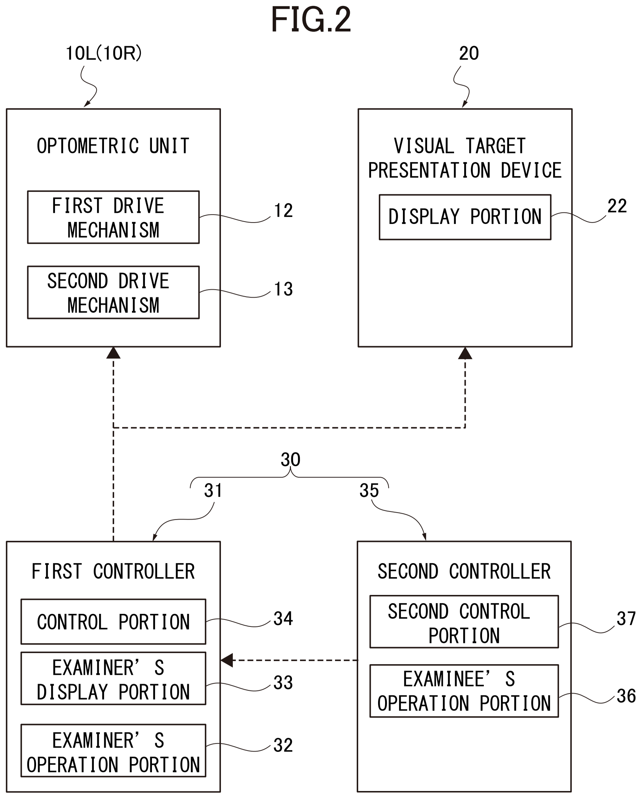

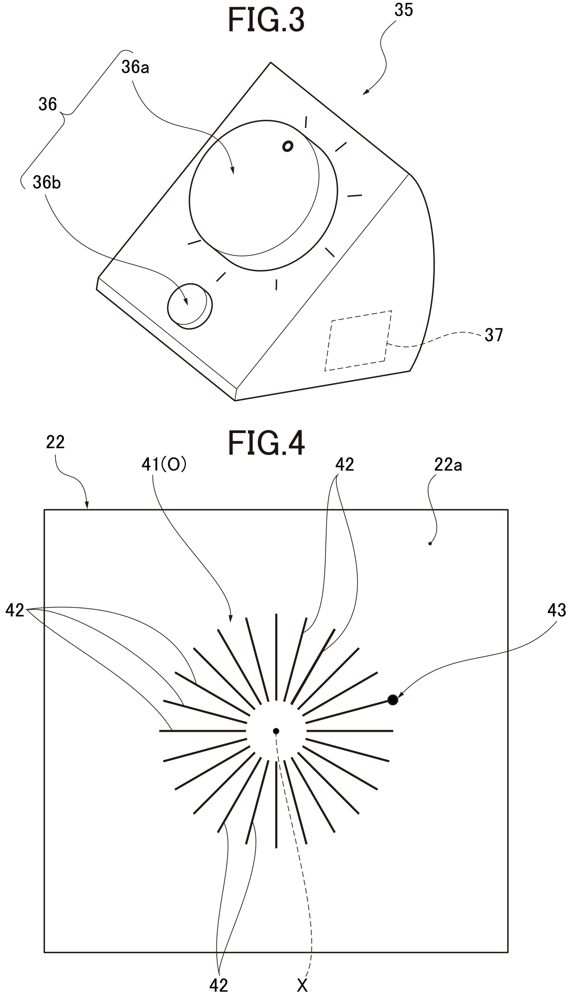

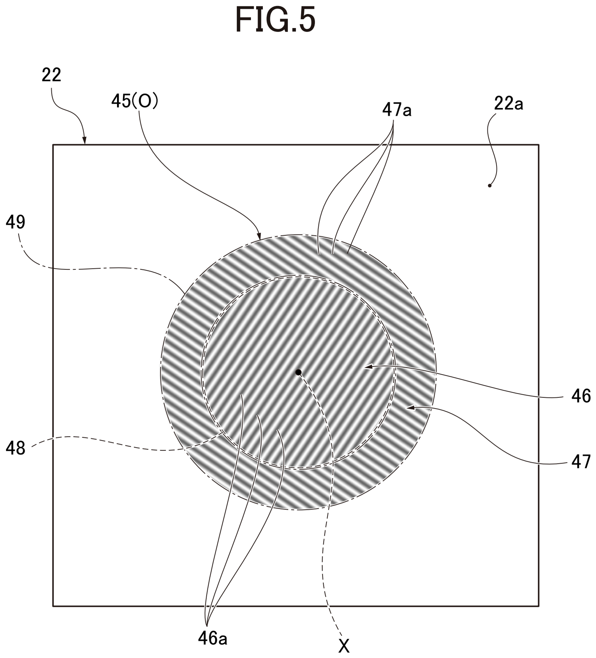

FIG. 1 is a schematic view of an ophthalmologic apparatus according to a first embodiment. FIG. 2 is a schematic view of a control system in the ophthalmologic apparatus of the first embodiment. FIG. 3 is a schematic perspective view of a second controller. FIG. 4 is a schematic view of a radial chart. FIG. 5 is a schematic view of an astigmatism test chart.

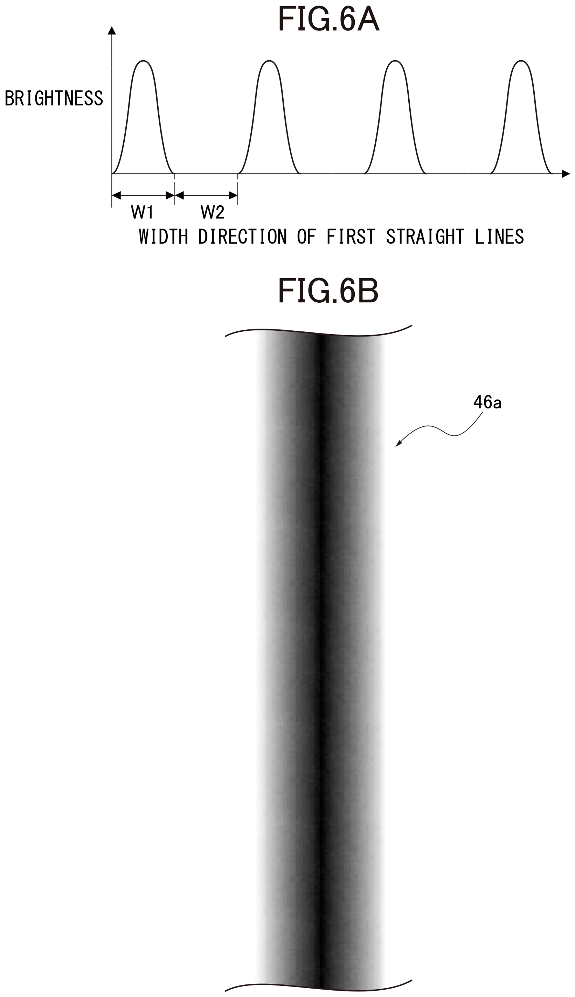

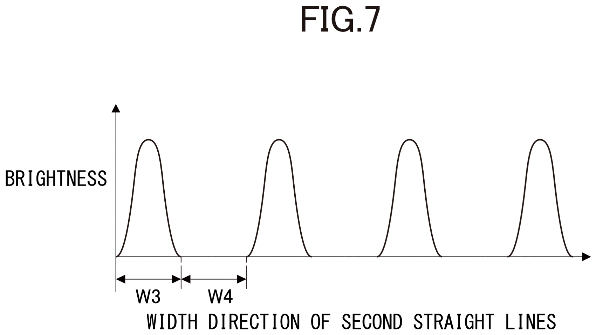

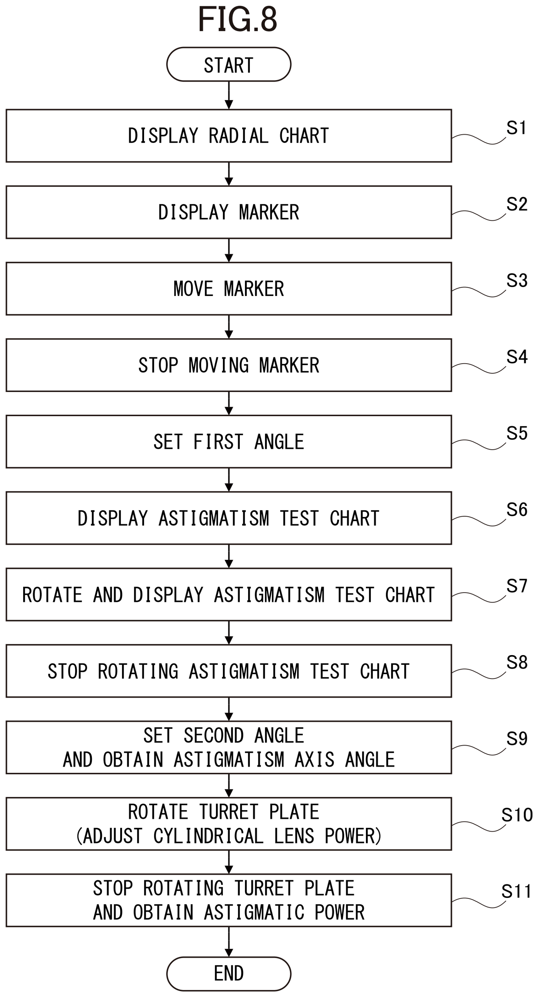

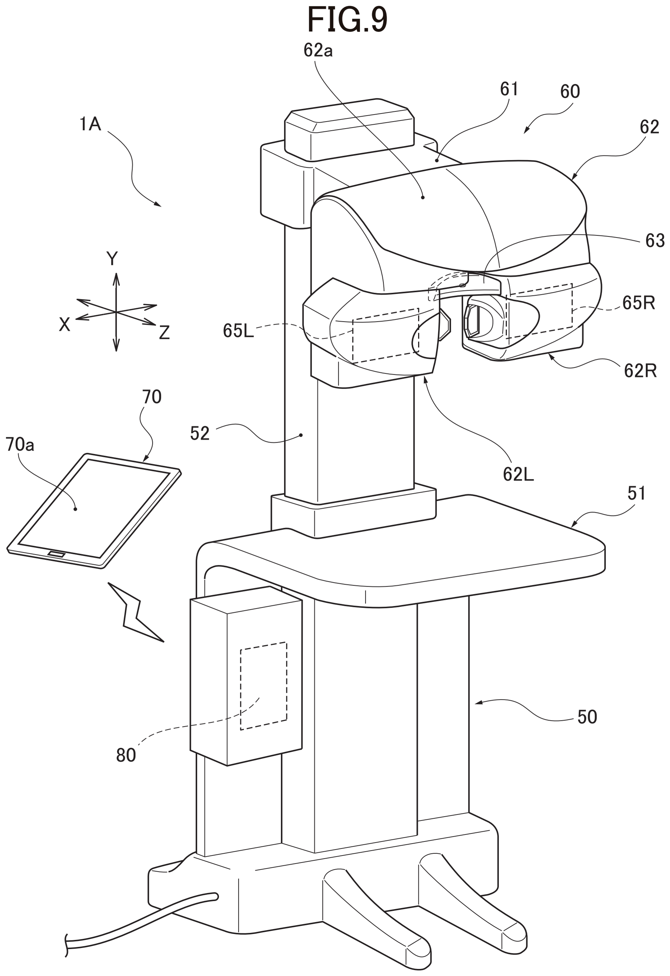

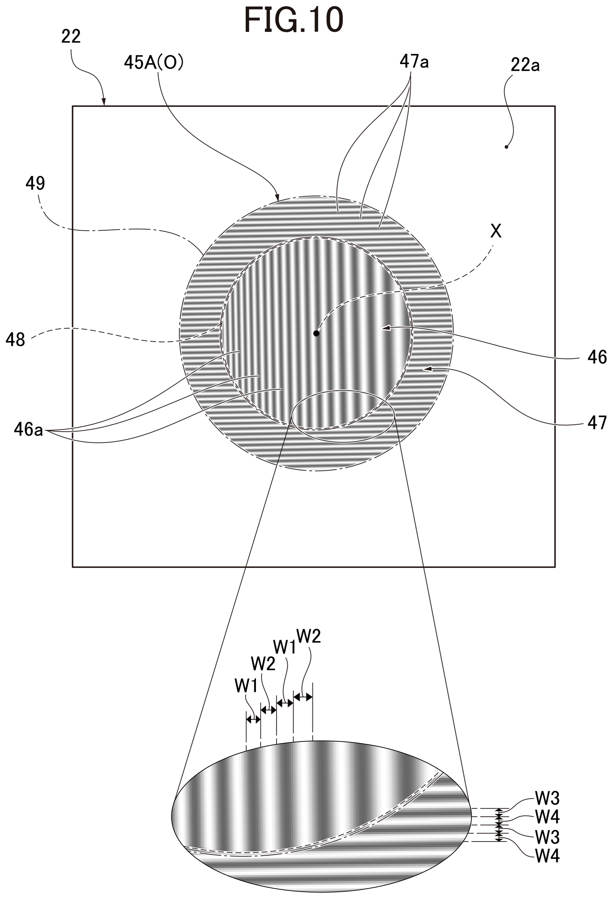

FIG. 6A is a schematic view showing brightness of a first visual target. FIG. 6B is an enlarged view of a portion of a first straight line. FIG. 7 is a schematic view showing brightness of a second visual target. FIG. 8 is a flowchart of an astigmatism test procedure using the ophthalmologic apparatus of the first embodiment. FIG. 9 is a perspective view of an ophthalmologic apparatus according to a second embodiment. FIG. 10 is a schematic view of an astigmatism test chart of a modified embodiment.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for the sake of clarity.

Embodiments for implementing the ophthalmologic apparatus and the method for controlling the ophthalmologic apparatus of the present invention will be described with reference to FIGS. 1 to 10. In the present specification, the X, Y, and Z axes are defined as shown in FIGS. 1 and 9. From the perspective of an examinee S facing the ophthalmologic apparatus, the X axis (X direction) represents the left-right direction, the Y axis (Y direction) represents the vertical direction (i.e., the up-down direction), and the Z axis (Z direction) represents the front-back direction orthogonal to both the X and Y axes.

The present invention is described with reference to a first embodiment. An ophthalmologic apparatus 1 according to the first embodiment enables at least astigmatism testing. The astigmatism test subjectively determines the axial angle of the astigmatic axis (astigmatic axis angle) and the degree of astigmatism of an examinee S (subject eye). Specifically, an examiner E presents to the examinee S an astigmatism test chart 45 (see FIG. 5), which serves as a visual target O for the astigmatism test, using the ophthalmologic apparatus 1, and obtains the astigmatic axis angle and the degree of astigmatism based on the examinee's responses to the astigmatism test chart 45.

Besides the astigmatism test, the ophthalmologic apparatus 1 may enable subjective tests such as the distance vision test, the near vision test, the contrast sensitivity test, the glare test, and the visual field test. Subjective tests obtain ocular characteristics based on responses of the examinee S viewing the visual target O. The distance vision test measures visual acuity at a distance. The near vision test measures visual acuity at a near position. The contrast sensitivity test measures the ability of the examinee S to distinguish subtle differences in brightness. The glare test evaluates the effect of reduced contrast in the retinal image of the examinee S, i.e., disability glare. The visual field test measures the extent and/or defects in the visual field of the examinee S.

As shown in FIG. 1, the ophthalmologic apparatus 1 includes a subjective optometric device 10, a visual target presentation device 20, and an operation device 30 (see FIG. 2).

The subjective optometric device 10 is arranged in front of the examinee S and configured to selectively place optical elements, including cylindrical lenses of different powers, in the optical path along the line of sight of the subject eye. In other words, the subjective optometric device 10 arranges the optical elements between the subject eye and the astigmatism test chart 45, which is described later. The subjective optometric device 10 is supported by a pillar 3 attached to the top panel of an optometric table 2 via a support arm 4.

The pillar 3 includes a cylindrical shaft 3a and a movable shaft 3b having a lower end supported by the cylindrical shaft 3a. The movable shaft 3b is movable in the Y direction by manual operation or by a drive mechanism not shown in the drawings. The drive mechanism may be, for example, a feed screw mechanism or a cylinder device driven by using a drive motor. The support arm 4 includes a first end attached to the movable shaft 3b and a second end extending obliquely upward. The support arm 4 is rotatable about the movable shaft 3b. The subjective optometric device 10 is suspended from the distal end of the support arm 4.

As shown in FIG. 1, the subjective optometric device 10 includes a pair of optometric units 10L, 10R, respectively corresponding to the left and right eyes of the examinee S. Each of the optometric units 10L, 10R includes a housing 11L, 11R. Each of the housings 11L, 11R houses a plurality of turret plates (not shown), a first drive mechanism 12 (see FIG. 2), and a second drive mechanism 13 (see FIG. 2).

Each of the housings 11L, 11R includes an optometric window LW, RW. The optometric windows LW, RW are openings penetrating the housings 11L, 11R. The examinee S can look through the optometric windows LW, RW, and view forward through the subjective optometric device 10.

The turret plates are circular plates of the same shape and are arranged in the thickness direction. Each of the turret plates is rotatably held inside the housings 11L, 11R. A plurality of optical elements is arranged on the same circumference of each turret plate. The optical elements include spherical lenses of different powers, rotary prisms, cross cylinders, wavefront modulators, and polarizing plates, in addition to cylindrical lenses of different powers. Certain optical elements, such as cylindrical lenses and cross cylinders, are rotatably mounted on the turret plates. Rotation of the cylindrical lens relative to the turret plate changes its axial direction. As each turret plate rotates, one of the optical elements is positioned to face the optometric window LW, RW.

The first drive mechanism 12 and the second drive mechanism 13 are, for example, pulse motors. The operation device 30 drives the first drive mechanism 12 with a control signal to independently rotate the turret plates. As a result, the subjective optometric device 10 selectively places one of the optical elements in the optical path along the line of sight of the subject eye. The operation device 30 drives the second drive mechanism 13 with a control signal to independently rotate the optical elements rotatably mounted on the turret plates. As a result, the subjective optometric device 10 adjusts, for example, the axial direction of the cylindrical lens to any orientation. In other words, in the ophthalmologic apparatus 1 of the first embodiment, the subjective optometric device 10 constitutes a measurement optical system including optical elements (cylindrical lenses) whose power and axial direction can be changed.

The visual target presentation device 20 is configured to present a visual target O, including the astigmatism test chart 45, in front of the subject eye. The visual target presentation device 20 includes a display housing 21 and a display portion 22.

The display housing 21 houses display portion 22 and the display portion 22 is visible through an opening 21a formed on the front surface of the display housing 21. The operation device 30 controls the display portion 22 with control signals to display the visual target O in a visual target presentation area 22a. The display portion 22 may be a liquid crystal display (LCD), an organic electroluminescence (EL) display, a plasma display, or the like.

The visual target presentation area 22a has a rectangular shape in one embodiment. The operation device 30 switches the visual target O displayed in the visual target presentation area 22a or changes its display state based on a control signal. The visual target O displayed in the visual target presentation area 22a may include, for example, the radial chart 41 described later, a Landolt ring, letters such as alphabetic characters, hiragana, or katakana, numbers, and an E-shaped visual target.

The operation device 30 includes a first controller 31 and a second controller 35.

The first controller 31 is an information processing device that is configured to send control signals to the optometric units 10L, 10R and the visual target presentation device 20. The first controller 31 may be, for example, a notebook computer, a desktop computer, or the like. The first controller 31 may be a controller dedicated to the ophthalmologic apparatus 1. The first controller 31 may also be a portable device carried by the examiner E, such as a tablet terminal or a smart phone. As shown in FIG. 2, the first controller 31 includes an examiner's operation portion 32, an examiner's display portion 33, and a control portion 34.

The examiner's operation portion 32 includes switches for optometric operations, a dial for a heterophoria test, and switches for a visual acuity test, among others. The examiner E operates the examiner's operation portion 32. The examiner's operation portion 32 is configured to receive the operations of the examiner E and send operation signals according to the operations received. The operation signals are sent to the control portion 34.

The examiner's display portion 33 is a touch panel display provided in the first controller 31 and includes a sending button. The examiner E may operate the sending button on the examiner's display portion 33 for a predetermined transmission. The operation device 30 controls the examiner's display portion 33 with control signals, and the examiner's display portion 33 displays information such as test information. The examiner's display portion 33 is configured to receive operations of the examiner E and send operation signals according to the operations received. The operation signals are sent to the control portion 34.

The control portion 34 is an information processing mechanism configured to execute various processes such as arithmetic and control processes. The control portion 34 includes a Central Processing Unit (CPU), a Graphics Processing Unit (GPU), an Application Specific Integrated Circuit (ASIC), a programmable logic device such as a Simple Programmable Logic Device (SPLD), a Complex Programmable Logic Device (CPLD), or a Field Programmable Gate Array (FPGA), a storage circuit, and a storage device. The control portion 34 sends predetermined control signals based on operation signals from the examiner's operation portion 32 or the examiner's display portion 33. The control portion 34 also sends predetermined control signals based on operation signals from the second control portion 37, which is described later. The control portion 34 sends the control signals to the first drive mechanism 12 and the second drive mechanism 13 of the optometric units 10L, 10R, to the display portion 22 of the visual target presentation device 20, and to the examiner's display portion 33. As a result, the control portion 34 integrally controls the optometric units 10L, 10R, the display portion 22 of the visual target presentation device 20, and the examiner's display portion 33.

The second controller 35 is a controller for the examinee S that is configured to receive operations of the examinee S and send predetermined operation signals to the control portion 34 of the first controller 31. The second controller 35 includes an examinee's operation portion 36 and a second control portion 37.

As shown in FIG. 3, the examinee's operation portion 36 includes a dial member 36a and a push button 36b. The dial member 36a has a cylindrical shape and rotates about a rotation shaft (not shown) provided at its center. The push button 36b is provided below the dial member 36a, and the examinee S pushes down the push button 36b.

The second control portion 37 sends operation signals according to the examinee's operations on the examinee's operation portion 36. Specifically, the second control portion 37 sends operation signals based on the rotation angle, rotation direction, and rotation speed of the dial member 36a, and on whether the push button 36b is pressed. The second control portion 37 sends these operation signals to the control portion 34.

The control portion 34 sends control signals to the display portion 22 and controls the display portion 22 of the visual target presentation device 20 to display the radial chart 41 or the astigmatism test chart 45. Furthermore, the control portion 34 controls the display portion 22 to display the astigmatism test chart 45 and then rotate it within the visual target presentation area 22a. Specifically, the astigmatism test chart 45 is first displayed in a preset state in the visual target presentation area 22a and then rotated within the visual target presentation area 22a about a rotation center point. The rotation angle and rotation speed of the astigmatism test chart 45 may be set as desired.

The radial chart 41 is a visual target O for obtaining an approximate astigmatic axis angle of the examinee S. As shown in FIG. 4, the radial chart 41 is a visual target O including a plurality of radial lines 42. The radial lines 42 are straight lines extending radially from a virtual center point X set at the center of the visual target presentation area 22a. The radial lines 42 extend in all radial directions at equal angular intervals of 15 degrees, dividing 360 degrees into 24 directions. The virtual center point X is not displayed.

The control portion 34 sends control signals to the display portion 22 to display the radial chart 41 in the visual target presentation device 20 and simultaneously displays a marker 43 in the vicinity of a distal end of one of the radial lines 42. "The vicinity of a distal end" refers to a predetermined position around the distal end of the radial line 42. The marker 43 may be placed at a position continuous with the radial line 42 or at a position separated from the distal end of the radial line 42. The marker 43 has, for example, a circular shape with a diameter slightly greater than the width of the radial line 42.

The control portion 34 sends control signals to the display portion 22 to sequentially move the marker 43 along the circumferential direction of the radial chart 41. Furthermore, the control portion 34 sends control signals to the display portion 22 to fix the marker 43 in the vicinity of the distal end of the radial line 42 that the examinee S clearly perceives based on the operation input from the examinee S through the examinee's operation portion 36.

The astigmatism test chart 45 is a visual target O for measuring the astigmatic axis angle and the degree of astigmatism of the examinee S. As shown in FIG. 5, the astigmatism test chart 45 includes a first visual target 46 and a second visual target 47.

The first visual target 46 includes a plurality of first straight lines 46a arranged in parallel at predetermined intervals. The first visual target 46 is displayed in a first area 48 defined in the visual target presentation area 22a. The first area 48 is a circular area in which the virtual center point X is defined at the center of the visual target presentation area 22a. In FIG. 5, the first area 48 is shown surrounded by a dashed line. The dashed line surrounding the first area 48 and the virtual center point X are not actually displayed. The size of the first area 48 may be set as desired.

As shown in FIG. 6A, each of the first straight lines 46a has brightness in its width direction that varies according to a sinusoidal function. In other words, the first visual target 46 exhibits a stripe pattern of spatial frequency. As a result, as shown in FIG. 6B, each of the first straight lines 46a has side edges in the width direction that are blurred. Widths W1 of the first straight lines 46a are the same. Furthermore, the interval W2 between two adjacent first straight lines 46a is set equal to the width W1.

The second visual target 47 includes a plurality of second straight lines 47a arranged in parallel at predetermined intervals and extending in a direction perpendicular to the first straight lines 46a. The second visual target 47 is displayed in a second area 49 defined in the visual target presentation area 22a. The second area 49 is an annular area surrounding the entire circumference of the first area 48 and has a constant width. In FIG. 5, the second area 49 is shown enclosed by a chain double-dashed line. However, the chain double-dashed line surrounding the second area 49 is not actually displayed. The width of the second area 49 may be set as desired, and the areal ratio between the first area 48 and the second area 49 may also be set as desired.

In this embodiment, the outer boundary of the first area 48 substantially coincides with the inner boundary of the second area 49. In other words, the gap between the first area 48 and the second area 49 is substantially absent. Accordingly, some of the first straight lines 46a constituting the first visual target 46 are substantially continuous at both ends with one of the second straight lines 47a constituting the second visual target 47.

As shown in FIG. 7, each of the second straight lines 47a has brightness in its width direction that varies according to a sinusoidal function. In other words, the second visual target 47 exhibits a stripe pattern of spatial frequency. As a result, similar to the first straight lines 46a, each of the second straight lines 47a has side edges in the width direction that are blurred (see FIG. 6B). The widths W3 of the second straight line 47a are the same. Furthermore, the width W3 of each second straight line 47a is set equal to the width W1 of the first straight lines 46a. Moreover, the interval W4 between adjacent second straight lines 47a is set equal to the width W3.

In other words, in the astigmatism test chart 45, the width W1 of the first straight lines 46a, the interval W2 between adjacent first straight lines 46a, the width W3 of the second straight lines 47a, and the interval W4 between adjacent second straight lines 47a are all set equal.

Furthermore, while viewing the astigmatism test chart 45, the examinee S can adjust at least one of the width W1 of the first straight lines 46a, the interval W2 between adjacent first straight lines 46a, the width W3 of the second straight lines 47a, and the interval W4 between adjacent second straight lines 47a.

In this case, for example, the display portion 22 stores in advance a plurality of first visual targets 46 with different spatial frequencies of stripe patterns and a plurality of second visual targets 47 with different spatial frequencies of stripe patterns. The second control portion 37 sends predetermined operation signals to the control portion 34 based on operations of the examinee S on the examinee's operation portion 36. The control portion 34 sends control signals to the display portion 22 based on the operation signals from the second control portion 37. The control portion 34 selects the first or second visual target 46, 47 having a stripe pattern of spatial frequency according to the operations of the examinee S and controls the display portion 22 to display the selected one. In this way, at least one of the width W1 of the first straight lines 46a, the interval W2 between adjacent first straight lines 46a, the width W3 of the second straight lines 47a, and the interval W4 between adjacent second straight lines 47a is changed according to the operations of the examinee S through the examinee's operation portion 36.

The control portion 34 sends control signals to the display portion 22 to stop the rotation of the astigmatism test chart 45 in a state where the examinee S clearly perceives the first visual target 46. Then, the control portion 34 sets a predetermined angle based on the inclination direction of the first visual target 46 when the rotation is stopped. The control portion 34 then sends control signals to the second drive mechanism 13 to rotate the cylindrical lenses relative to the turret plate so as to align the axial directions of all cylindrical lenses of the subjective optometric device 10 with the predetermined angle. Furthermore, the control portion 34 sends control signals to the first drive mechanism 12 to rotate the turret plate, to which the plurality of cylindrical lenses is attached, so as to change the power of the cylindrical lenses until the examinee S clearly perceives the second visual target 47. In other words, the control portion 34 places the cylindrical lens that enables the examinee S to clearly perceive the second visual target 47 between the subject eye and the astigmatism test chart 45.

FIG. 8 shows an example of the flow of an astigmatism test using the ophthalmologic apparatus 1 of the first embodiment. The astigmatism test starts when the examiner E operates the first controller 31.

In Step S1, the control portion 34 sends control signals to the visual target presentation device 20 based on the operation signals from the first controller 31. The control portion 34 then controls the display portion 22 to display the radial chart 41. The display portion 22 displays the radial chart 41 at the center of the visual target presentation area 22a. The process then proceeds to Step S2. The examinee S views the radial chart 41.

In Step S2, the control portion 34 sends control signals to the visual target presentation device 20 and controls the display portion 22 to display the marker 43. The display portion 22 displays the marker 43 in the vicinity of the distal end of a predetermined one of the radial lines 42. The process then proceeds to Step S3. The examinee S views the marker 43.

In Step S3, the control portion 34 sends control signals to the visual target presentation device 20. The control portion 34 sequentially moves the marker 43 along the circumferential direction of the radial chart 41. The timing of moving the marker 43 may be preset or may be set based on operations of the examiner E on the examiner's operation portion 32. Furthermore, the control portion 34 may move the marker 43 based on operations of the examinee S on the examinee's operation portion 36. The movement direction of the marker 43 may be preset or may be determined based on operations of the examiner E on the examiner's operation portion 32 or of the examinee S on the examinee's operation portion 36. The process then proceeds to Step S4.

At this point, the examiner E asks the examinee S which radial line 42 the examinee S sees most clearly. When the examiner E receives a response from the examinee S that all the radial lines 42 appear uniform, the examiner E stops the astigmatism test on the assumption that the examinee S has no astigmatism.

In Step S4, the examiner's operation portion 32 receives an operation of the examiner E to stop the movement of the marker 43 in the vicinity of the distal end of the radial line 42 that the examinee S sees most clearly. The examiner E operates the examiner's operation portion 32 based on the response of the examinee S who has viewed the radial chart 41 and the marker 43. The examiner's operation portion 32 sends operation signals to the control portion 34 according to the received operation. The control portion 34 then sends control signals to the visual target presentation device 20 according to the operation signals and stops the movement of the marker 43. The process then proceeds to Step S5.

In Step S5, the control portion 34 sets the inclination direction of the radial line 42 located in the vicinity of the marker 43, where the movement has been stopped, as a first angle. This radial line 42 located in the vicinity of the stopped marker 43 is the one that the examinee S sees most clearly. The process then proceeds to Step S6.

In Step S6, the examiner's operation portion 32 receives an operation of the examiner E to switch the visual target O displayed on the display portion 22 from the radial chart 41 to the astigmatism test chart 45. The examiner's operation portion 32 sends operation signals to the control portion 34 according to the received operation. The control portion 34 then sends control signals to the visual target presentation device 20 according to the operation signals to display the astigmatism test chart 45 on the display portion 22. In other words, Step S6 corresponds to a step for displaying the astigmatism test chart 45 on the display portion 22.

The astigmatism test chart 45 is displayed at the center of the visual target presentation area 22a. The control portion 34 displays the astigmatism test chart 45 with the first straight lines 46a aligned along the first angle set in Step S5. As a result, the rotation of the astigmatism test chart 45 starts from a position where the first straight lines 46a are aligned with the first angle. The examinee S views the astigmatism test chart 45. The process then proceeds to Step S7.

In Step S7, the dial member 36a receives an operation of the examinee S who views the astigmatism test chart 45. In other words, the examinee S views the astigmatism test chart 45 to simultaneously compare the first and second visual targets 46 and 47 (hereinafter referred to as "two groups of straight lines"). The examinee S then rotates the dial member 36a of the examinee's operation portion 36 based on his or her perception of the astigmatism test chart 45. At this time, the examinee S adjusts the rotation angle and/or the rotation direction of the dial member 36a so that the first visual target 46 appears clearest while the second visual target 47 appears blurriest.

The second control portion 37 sends operation signals to the control portion 34 according to the operation received by the dial member 36a, that is, the rotation angle and/or rotation direction of the dial member 36a. The control portion 34 sends control signals to the visual target presentation device 20 according to the operation signals to rotate the astigmatism test chart 45 about the virtual center point X. In other words, the astigmatism test chart 45 rotates based on operations of the examinee S on the second controller 35. The process then proceeds to Step S8. Step S7 corresponds to a step for displaying and rotating the astigmatism test chart 45 on the display portion 22.

At this point, the control portion 34 may change the width W1 of the first straight lines 46a or the interval W2 between adjacent first straight lines 46a based on operations of the examinee S on the examinee's operation portion 36. Furthermore, the control portion 34 may change the width W3 of the second straight lines 47a or the interval W4 between adjacent second straight lines 47a based on operations of the examinee S on the examinee's operation portion 36. In other words, Step S7 corresponds to a step for changing at least one of the width W1 of the first straight lines 46a, the interval W2 between adjacent first straight lines 46a, the width W3 of the second straight lines 47a, and the interval W4 between adjacent second straight lines 47a.

In Step S8, the push button 36b receives an operation of the examinee S. In other words, the examinee S presses the push button 36b at the timing when he or she perceives the first visual target 46 most clearly while viewing the astigmatism test chart 45. The second control portion 37 sends operation signals to the control portion 34 when the push button 36b is pressed. Upon receiving the operation signals, the control portion 34 sends control signals to the visual target presentation device 20 to stop the rotation of the astigmatism test chart 45. Step S8 corresponds to a step for stopping the rotation of the astigmatism test chart 45 when the examinee S sees the first visual target 46 most clearly.

As a result, the astigmatism test chart 45 is displayed in a state where the examinee S sees the first visual target 46 most clearly. When the examinee S sees the first visual target 46 most clearly, the second visual target 47 appears blurriest. In other words, the examinee S presses the push button 36b when he or she perceives that the visual difference between the first and second visual targets 46 and 47 is greatest. The process then proceeds to Step S9.

In Step S9, the control portion 34 detects the angle of the inclination direction of the first straight lines 46a of the astigmatism test chart 45 where the rotation has been stopped and sets the detected angle as the second angle (predetermined angle). The first straight lines 46a of the astigmatism test chart 45 where the rotation has been stopped correspond to the first visual target 46 in a state where the examinee S sees them most clearly. The angle of the inclination direction of the first straight lines 46a refers to the inclination angle of the first straight lines 46a relative to the horizontal or vertical direction set in the visual target presentation area 22a.

The control portion 34 sends control signals to the second drive mechanism 13 to rotate each cylindrical lens relative to the turret plate and to align the axial directions of the cylindrical lenses with the second angle. Furthermore, the control portion 34 obtains the astigmatism axial angle of the subject eye based on the second angle. Then, the process proceeds to Step S10. Step S9 corresponds to a step for aligning the axial directions of the cylindrical lenses (optical elements) with the second angle (predetermined angle) set based on the inclination direction of the first visual target 46 where the rotation has been stopped.

In Step S10, the dial member 36a receives an operation of the examinee S who views the astigmatism test chart 45 again. In other words, the examinee S views the astigmatism test chart 45, where the rotation has been stopped, to visually compare the two different groups of straight lines simultaneously. Then, the examinee S rotates the dial member 36a of the examinee's operation portion 36 based on his or her perception of the astigmatism test chart 45. At this point, the examinee S adjusts the rotation angle and/or the rotation direction of the dial member 36a so as to see the first and second visual targets 46 and 47 to the same degree. Seeing the first and second visual targets 46 and 47 to the same degree means that the examinee S clearly sees both visual targets 46, 47.

The second control portion 37 sends operation signals to the control portion 34 according to the rotation angle and/or the rotation direction of the dial member 36a. The control portion 34 sends control signals to the first drive mechanism 12 according to the operation signals. The control portion 34 rotates the turret plate so that one of the cylindrical lenses faces the optometric window LW, RW. This adjusts the power of the cylindrical lens arranged in the optical path in the eye direction of the subject eye. Then, the process proceeds to Step S11.

In Step S11, the push button 36b receives an operation by the examinee S. In other words, the examinee S presses the push button 36b when he or she perceives the first and second visual targets 46 and 47 equally based on his or her perception of the astigmatism test chart 45. When the push button 36b is pressed, the second control portion 37 sends operation signals to the control portion 34. Upon receiving the operation signals, the control portion 34 sends control signals to the first drive mechanism 12 to stop the rotation of the turret plate. The control portion 34 obtains the degree of astigmatism of the subject eye based on the power of the cylindrical lens facing the optometric window LW, RW. The process then proceeds to the END. Steps S10 and S11 correspond to a step for changing the power of the cylindrical lens (optical element) while its axial direction is aligned with the second angle (predetermined angle) so that the examinee S clearly sees the second visual target 47.

The effects of the ophthalmologic apparatus 1 of the first embodiment are described below.

The ophthalmologic apparatus 1 includes a display portion 22 configured to display an astigmatism test chart 45 for obtaining the astigmatic axis angle of the subject eye, and a control portion 34 configured to control the display portion 22. The control portion 34 controls the display portion 22 to display and rotate the astigmatism test chart 45 within the visual target presentation area 22a. The astigmatism test chart 45 includes a first visual target 46 and a second visual target 47. The first visual target 46 includes a plurality of first straight lines 46a arranged in parallel with each other at predetermined intervals. The second visual target 47 includes a plurality of second straight lines 47a arranged in parallel with each other at predetermined intervals and extending in a direction orthogonal to the first straight lines 46a. The first visual target 46 is displayed in a circular first area 48, and the second visual target 47 is displayed in an annular second area 49 surrounding the first area 48.

A method for controlling the ophthalmologic apparatus 1 includes a step (Step S6) for displaying the astigmatism test chart 45 on the display portion 22. The astigmatism test chart 45 includes a first visual target 46 including a plurality of first straight lines 46a arranged in parallel with each other at predetermined intervals, and a second visual target 47 including a plurality of second straight lines 47a arranged in parallel with each other at predetermined intervals and extending in a direction orthogonal to the first straight lines 46a.

The ophthalmologic apparatus 1 allows the examinee S to compare the central portion and the surrounding portion of the astigmatism test chart 45, thereby enabling a simultaneous visual comparison between two groups of straight lines extending in mutually orthogonal directions. This makes it easier for the examinee S to recognize the visual difference between the first visual target 46 and the second visual target 47 when viewing the astigmatism test chart 45. Therefore, the ophthalmologic apparatus 1 facilitates simultaneous visual comparison between the two groups of straight lines extending in the mutually orthogonal directions.

The degree of the visual difference between the two groups of straight lines varies among examinees depending on the stripe pattern of the first visual target 46 or the second visual target 47. In other words, each examinee S has a stripe pattern in which the visual difference is more readily noticeable.

The ophthalmologic apparatus 1 further includes the second controller 35 configured to receive an operation of the examinee S and send a predetermined operation signal to the control portion 34. The control portion 34 changes at least one of the width W1 of the first straight lines 46a, the interval W2 between adjacent first straight lines 46a, the width W3 of the second straight lines 47a, and the interval W4 between adjacent second straight lines 47a based on the operation received by the second controller 35.

The method for controlling the ophthalmologic apparatus 1 further includes a step (Step S7) for changing at least one of the width W1 of the first straight lines 46a, the interval W2 between adjacent first straight lines 46a, the width W3 of the second straight lines 47a, and the interval W4 between adjacent second straight lines 47a based on the operation of the examinee S on the second controller 35.

As a result, the ophthalmologic apparatus 1 changes at least one of the width W1, the interval W2, the width W3, and the interval W4 based on the examinee's own instructions according to his or her perception. In other words, the examinee S can change the stripe pattern of the first or second visual target 46, 47 by his or her own instructions into a state where the visual difference is more easily recognized. Accordingly, the ophthalmologic apparatus 1 further facilitates simultaneous visual comparison between the two groups of straight lines.

When the outlines of the first and second straight lines 46a, 47a are clear, the first and second straight lines 46a, 47a are displayed in a single color. Thus, it is difficult for the examinee S to recognize the subtle difference between a state where the first or second visual target 46, 47 is clearly seen and a state where it is not.

In contrast, in the astigmatism test chart 45, the outlines of the first and second straight lines 46a, 47a are blurred. This allows the ophthalmologic apparatus 1 to partially change the color tone of the first and second straight lines 46a, 47a. Thus, when the examinee S with astigmatism views the astigmatism test chart 45, the examinee S can more easily recognize the subtle difference between the state where the first or second visual target 46, 47 is clearly seen and the state where it is not. Accordingly, the ophthalmologic apparatus 1 facilitates fine adjustment when rotating the astigmatism test chart 45.

In particular, in the first embodiment, the astigmatism test chart 45 is configured such that brightness in the width direction of the first and second straight lines 46a, 47a changes according to a sinusoidal function. As a result, the first and second visual targets 46, 47 form a stripe pattern in which brightness repeatedly changes at constant intervals, a so-called spatial frequency stripe pattern. Thus, the ophthalmologic apparatus 1 enables the examinee S to more easily recognize the subtle difference between the state where the first or second visual target 46, 47 is clearly seen and the state where it is not.

The ophthalmologic apparatus 1 further includes the subjective optometric device 10 that is arranged between the subject eye and the astigmatism test chart 45 and that includes a plurality of cylindrical lenses as optical elements configured to change their powers and axial directions. The control portion 34 controls the display portion 22 to display and rotate the astigmatism test chart 45 in the visual target presentation area 22a of the display portion 22. The control portion 34 stops the rotation of the astigmatism test chart 45 when the examinee S clearly sees the first visual target 46 based on the operation of the examinee S. Then, the control portion 34 controls the second drive mechanism 13 of the subjective optometric device 10 to set the axial directions of the cylindrical lenses at the second angle set based on the inclination direction of the first visual target 46 when the rotation of the astigmatism test chart 45 is stopped. Furthermore, the control portion 34 controls the first drive mechanism 12 of the subjective optometric device 10 to change the power of the cylindrical lens such that the examinee S clearly sees the second visual target 47.

In other words, the method for controlling the ophthalmologic apparatus 1 further includes the displaying and rotating step (Step S7), the stopping step (Step S8), the axial direction adjusting step (Step S9), and the power adjusting step (Steps S10 and S11). In the displaying and rotating step, the astigmatism test chart 45 is displayed and rotated on the display portion 22. In the stopping step, the rotation of the astigmatism test chart 45 is stopped when the examinee S clearly sees the first visual target 46. In the axial direction adjusting step, the axial directions of the cylindrical lenses are aligned with the second angle set based on the inclination direction of the first visual target 46 in a state that the rotation is stopped. In the power adjusting step, the power of the cylindrical lens is changed in a state that the axial direction of the cylindrical lens is aligned with the second angle, so that the examinee S clearly sees the second visual target 47.

This allows the ophthalmologic apparatus 1 to obtain the astigmatic axis angle and the degree of astigmatism of the examinee S with high accuracy.

The control portion 34 controls the display portion 22 to display the radial chart 41 including a plurality of radial lines 42 extending in a radial direction. The control portion 34 controls the display portion 22 to display the astigmatism test chart 45 in a state where the first straight lines 46a are along the radial line 42 (first angle) that the examinee S clearly sees. In other words, the control portion 34 starts the rotation of the astigmatism test chart 45 from a position where the first straight lines 46a are along the radial line 42 that the examinee S clearly sees.

The ophthalmologic apparatus 1 can align the direction of the first straight lines 46a with the approximate direction in which the examinee S can clearly see them when displaying the astigmatism test chart 45. This enables the ophthalmologic apparatus 1 to determine the astigmatic axis angle without greatly rotating the astigmatism test chart 45.

The control portion 34 controls the display portion 22 to simultaneously display the radial chart 41 and the marker 43 in the vicinity of the distal end of one of the radial lines 42. The control portion 34 further controls the display portion 22 to sequentially move the marker 43. Furthermore, the control portion 34 controls the display portion 22 to fix the position of the marker 43 in the vicinity of the distal end of the radial line 42 that the examinee S clearly sees.

As a result, the ophthalmologic apparatus 1 enables the examiner E and the control portion 34 to easily determine the radial line 42 that the examinee S clearly sees.

The ophthalmologic apparatus 1 further includes the second controller 35 including the dial member 36a that is rotated by the examinee S. The control portion 34 controls the display portion 22 and the subjective optometric device 10 according to the rotation angle of the dial member 36a.

As a result, according to the operation of the dial member 36a by the examinee S, the ophthalmologic apparatus 1 can adjust the rotation of the astigmatism test chart 45 and the switching of the cylindrical lenses. In other words, the examinee S can intuitively rotate the astigmatism test chart 45 and switch the cylindrical lenses. Therefore, the ophthalmologic apparatus 1 can more accurately obtain the astigmatic axis angle and the degree of astigmatism of the examinee S.

The present invention is described with reference to a second embodiment. An ophthalmologic apparatus 1A according to the second embodiment shown in FIG. 9 is configured for subjective examinations including an astigmatism test, and objective examinations. In the objective examinations, the ophthalmologic apparatus 1A projects light onto the subject eye and obtains ocular characteristics based on the detection results of the reflected light. The objective examinations include measurement to obtain ocular characteristics and imaging to capture images of the subject eye. The objective examinations further include objective refractive measurement (refraction measurement), corneal shape measurement (keratometry), intraocular pressure measurement, fundus imaging, and measurement using optical coherence tomography (OCT).

The ophthalmologic apparatus 1A is a binocular-open type apparatus that is capable of simultaneously measuring ocular characteristics of both eyes while the examinee S keeps both eyes open. The ophthalmologic apparatus 1A may also measure ocular characteristics of each eye individually by shielding one of the eyes or turning off a fixation target.

As shown in FIG. 9, the ophthalmologic apparatus 1A includes a support base 50, a measurement unit 60, an examiner's controller 70, a control portion 80, and an examinee's controller (not shown).

The support base 50 includes an optometric table 51 placed on the floor, and a column 52 extending from the optometric table 51. The optometric table 51 may be adjustable in the Y direction (height position).

The measurement unit 60 includes an arm 61, a measurement head 62, and a forehead support 63. One end of the arm 61 is supported by the column 52, and the other end extends from the column 52 toward the near side (examinee's side) in the Z direction. The measurement head 62 is attached to a distal end of the arm 61. Thus, the measurement head 62 is suspended from the column 52 through the arm 61 above the optometric table 51. The arm 61 is movable in the Y direction relative to the column 52. The arm 61 may also be movable in the X direction and the Z direction relative to the column 52.

The measurement head 62 measures ocular characteristics of the subject eyes. The measurement head 62 includes a drive portion 62a and a pair of left and right measurement portions 62L, 62R provided below the drive portion 62a.

The drive portion 62a is configured to individually drive the left and right measurement portions 62L, 62R to move them horizontally (in the X direction and/or Z direction), vertically (in the Y direction), and to rotate them about the X and Y directions.

The left and right measurement portions 62L, 62R are provided as a pair, respectively corresponding to the left and right eyes of the examinee S. The left measurement portion 62L includes a left measurement optical system 65L for measuring ocular characteristics of the left eye of the examinee S. The right measurement portion 62R includes a right measurement optical system 65R for measuring ocular characteristics of the right eye of the examinee S. Measurement results obtained by the measurement head 62 are sent to the control portion 80.

Each of the left and right measurement optical systems 65L, 65R includes a plurality of optical systems, such as an anterior segment observation system, a visual target projection system, and a measurement optical system. The anterior segment observation system is an optical system for observing an anterior segment of the subject eye. The visual target projection system is an optical system for presenting various visual targets O, including the astigmatism test chart 45 (see FIG. 5) and the radial chart 41 (see FIG. 4). The measurement optical system is an optical system including optical elements whose powers and axial directions are changeable.

The control portion 80 is an information processing device provided below the optometric table 51. The control portion 80 is configured to send predetermined control signals to the measurement unit 60 including the left and right measurement optical systems 65L, 65R based on operation signals from the examiner's controller 70 or the examinee's controller. The control portion 80 is also configured to send to the examiner's controller 70 the measurement results of the ocular characteristics of the subject eye obtained by the measurement head 62. Thus, the control portion 80 comprehensively controls each part of the measurement unit 60 including the measurement optical systems, the visual target projection systems, and other related systems.

The examiner's controller 70 is configured to receive operations of the examiner E and send predetermined operation signals to the control portion 80. The examiner's controller 70 may be, for example, a tablet terminal and includes a display screen 70a. The examiner's controller 70 has a configuration similar to the examiner's operation portion 32 and the examiner's display portion 33 of the first controller 31 in the first embodiment. Thus, explanation of the examiner's controller 70 is not provided here.

The examinee's controller (not shown) is configured to receive operations of the examinee S and send predetermined operation signals to the control portion 80. The examinee's controller has a configuration similar to that of the second controller 35 in the first embodiment. Thus, explanation of the examinee's controller is not provided here.

The radial chart 41 and the astigmatism test chart 45 displayed in the ophthalmologic apparatus 1A are the same as those in the first embodiment. Thus, explanations of the charts 41, 45 are not provided here.

In other words, the ophthalmologic apparatus 1A of the second embodiment integrally includes the subjective optometric device 10, the visual target presentation device 20, and the control portion 34 of the operation device 30, as in the ophthalmologic apparatus 1 of the first embodiment.

The ophthalmologic apparatus of the present invention has been described with reference to the first and second embodiments. However, the specific configurations are not limited to those of these embodiments. Design changes, additions, and modifications are permitted as long as they do not depart from the scope of the invention as defined in the claims.

In the astigmatism test chart 45, the width W1 of the first straight lines 46a, the interval W2 between adjacent first straight lines 46a, the width W3 of the second straight lines 47a, and the interval W4 between adjacent second straight lines 47a are set to the same value. However, the width W1 of the first straight lines 46a or the interval W2 between adjacent first straight lines 46a are not necessarily set to the same value.

For example, as in the astigmatism test chart 45A of the modified embodiment shown in FIG. 10, the widths W1 of the first straight lines 46a may differ from one another. The intervals W2 between adjacent first straight lines 46a may also differ from one another.

In the astigmatism test chart 45A of the modified embodiment, the width W3 of the second straight lines 47a and the interval W4 between adjacent second straight lines 47a are set to the same value. However, the widths W3 of the second straight lines 47a and/or the intervals W4 between adjacent second straight lines 47a may differ from one another.

In the astigmatism test chart 45A of the modified embodiment, the widths W1 of the first straight lines 46a are set to become longer from one side to the other in the alignment direction of the first straight lines 46a. The intervals W2 between adjacent first straight lines 46a are also set to become longer from one side to the other in the alignment direction. However, the widths W1 of the first straight lines 46a and the intervals W2 between adjacent first straight lines 46a can be set to any value. Thus, the widths W1 and the intervals W2 are not always required to become longer from one side to the other in the alignment direction.

During the astigmatism test in the ophthalmologic apparatus 1, in Step S6, the astigmatism test chart 45 is displayed in a state where the first straight lines 46a are along the first angle set in Step S5. In other words, the astigmatism test chart 45 is displayed in a state that allows the rotation angle to be immediately adjusted. However, the procedure of displaying the astigmatism test chart 45 is not limited to the above.

In Step S6 for displaying and rotating the astigmatism test chart 45, the control portion 34 may perform a second phase after a first phase. In the first phase, the astigmatism test chart 45 is rotated 360 degrees. In the second phase, the rotation angle of the astigmatism test chart 45 is adjusted based on the examinee's perception of the astigmatism test chart 45.

In other words, the control portion 34 first displays the astigmatism test chart 45 in the center of the visual target presentation area 22a. At this time, the astigmatism test chart 45 is displayed in a state where the first straight lines 46a are along the first angle. Next, the control portion 34 automatically rotates the astigmatism test chart 45, which has been displayed in the visual target presentation area 22a, about the virtual center point X by 360 degrees (first phase). The rotation direction and rotation speed of the astigmatism test chart 45 in the first phase may be set as desired.

Then, the control portion 34 temporarily stops the rotation of the astigmatism test chart 45 when it rotates 360 degrees. At this time, the astigmatism test chart 45 is in a state where the first straight lines 46a are along the first angle. Then, the control portion 34 rotates the astigmatism test chart 45 according to the examinee's operation on the second controller 35 (second phase).

When the examinee S compares the two different groups of straight lines simultaneously, the examinee S may not notice the visual change of the first or second visual target 46, 47 with the rotation of the astigmatism test chart 45 if the rotation angle is too small or the rotation speed is too slow. Thus, the ophthalmologic apparatus 1 performs the first phase prior to the second phase, so that the examinee S can notice that the rotation of the astigmatism test chart 45 causes a visual change of the first or second visual target 46, 47, that is, a difference in the rotation angle causes a visual difference of the first or second visual target 46, 47.

Thus, the examinee S can more easily recognize the visual change of the first or second visual target 46, 47 with the rotation of the astigmatism test chart 45. This allows the ophthalmologic apparatus 1 to accurately obtain the axial angle of the astigmatic axis.

The ophthalmologic apparatus 1 controls the display portion 22 to display the radial chart 41. Then, the ophthalmologic apparatus 1 starts the rotation of the astigmatism test chart 45 from a position where the first straight lines 46a are along the first angle (radial line 42 that the examinee S has most clearly seen). However, the display portion 22 is not always required to display the radial chart 41. In other words, the ophthalmologic apparatus 1 may display at least the astigmatism test chart 45.

The ophthalmologic apparatus 1 adjusts the power of the cylindrical lenses in a state where the astigmatism test chart 45 is displayed such that the first and second visual targets 46, 47 are seen to the same degree. In other words, the ophthalmologic apparatus 1 adjusts the power of the cylindrical lens while the examinee S views the astigmatism test chart 45, thereby obtaining the degree of astigmatism. However, the visual target O to be seen by the examinee S when the ophthalmologic apparatus 1 adjusts the power of the cylindrical lenses is not limited to the astigmatism test chart 45.

The ophthalmologic apparatus 1 may adjust the power of the cylindrical lenses, for example, so that all the radial lines 42 are seen to the same degree when the display portion 22 displays the radial chart 41. In other words, the astigmatism test chart 45 may not be used when obtaining the degree of astigmatism.

In the astigmatism test chart 45, the brightness in the width direction of each first straight line 46a and second straight line 47a changes according to a sinusoidal function, which makes their outlines blurred. The brightness of the first and second straight lines 46a, 47a is not necessarily changed according to a sinusoidal function to make the outlines of the first and second straight lines 46a, 47a blurred. The brightness at both side edges in the width direction may be set higher than that at the middle portion. This makes the first and second straight lines 46a, 47a have blurred outlines.

A part of the first and second straight lines 46a, 47a may have blurred outlines. In other words, the first visual target 46 may include straight lines with clear outlines and straight lines with blurred outlines. The second visual target 47 may also include straight lines with clear outlines and straight lines with blurred outlines. Furthermore, all of the first and second straight lines 46a, 47a may be straight lines with clear outlines. The straight lines with clear outlines refer to straight lines in which brightness does not change in the width direction, that is, brightness is constant throughout.

In the astigmatism test chart 45, the outer boundary of the first area 48 substantially matches with the inner boundary of the second area 49, and a part of the first straight lines 46a is substantially continuous at both ends with any of the second straight lines 47a. However, it is only required that the second area 49 surrounds the entire circumference of the first area 48. Thus, a gap without a visual target O may be present between the first and second areas 48, 49. In this case, the first and second straight lines 46a, 47a become discontinuous.

The examinee's operation portion 36 of the second controller 35 includes the dial member 36a and the push button 36b. However, it is only required that the examinee's operation portion 36 receives an operation by the examinee S and sends operation signals according to the received operation. Thus, the examinee's operation portion 36 is not always required to include the dial member 36a.

The present invention is further described with respect to the above embodiments and modifications as follows:
(1) An ophthalmologic apparatus including:
   a display portion that is configured to display an astigmatism test chart for obtaining an astigmatism axis angle of an examinee, and
   a control portion that is configured to control the display portion to display and rotate the astigmatism test chart on the display portion,
   wherein the astigmatism test chart includes:
      a first visual target including a plurality of first straight lines that are arranged in parallel with each other at a predetermined interval, and
      a second visual target including a plurality of second straight lines that are arranged in parallel with each other at a predetermined interval and extend in a direction orthogonal to a direction of the first straight lines,
      wherein the first visual target is displayed in a circular first area, and the second visual target is displayed in an annular second area surrounding the first area.
(2) The ophthalmologic apparatus according to above (1), further including an examinee's controller that is configured to receive an operation of the examinee,
   wherein the control portion is configured to change at least one of a width of the first straight lines, an interval between the first straight lines, a width of the second straight lines, and an interval between the second straight lines based on the operation of the examinee on the examinee's controller.
(3) The ophthalmologic apparatus according to above (1) or (2), wherein at least a part of an outline of the first and second straight lines of the astigmatism test chart is blurred.
(4) The ophthalmologic apparatus according to above (3), wherein brightness in a width direction of at least a part of the first and second straight lines in the astigmatism test chart changes according to a sinusoidal function.
(5) The ophthalmologic apparatus according to any one of above (1) to (4), further including a measurement optical system arranged between a subject eye of the examinee and the astigmatism test chart, the measurement optical system including an optical element whose power and axial direction are changeable,
   wherein the control portion is configured to control the display portion to display and rotate the astigmatism test chart on the display portion and to stop rotation of the astigmatism test chart when the examinee clearly sees the first visual target, and
   wherein the control portion is configured to control the measurement optical system to set the axial direction of the optical element at a predetermined angle set based on an inclination direction of the first visual target in a state where the rotation of the astigmatism test chart is stopped, and to change the power of the optical element so that the examinee clearly sees the second visual target.
(6) The ophthalmologic apparatus according to any one of above (1) to (5), wherein the control portion is configured to control the display portion to display a radial chart including a plurality of radial lines extending in a radial direction, and
   wherein the control portion is configured to start rotation of the astigmatism test chart from a position where the first straight lines are along the radial line clearly seen by the examinee.
(7) The ophthalmologic apparatus according to above (6), wherein the control portion is configured to control the display portion to simultaneously display the radial chart and a marker in vicinity of a distal end of one of the radial lines of the radial chart, and
   wherein the control portion is configured to control the display portion to sequentially move the marker and then fix the marker in vicinity of a distal end of the radial line that is clearly seen by the examinee.
(8) The ophthalmologic apparatus according to any one of above (1) to (7), wherein, when the display portion displays and rotates the astigmatism test chart, the control portion is configured to perform a first phase in which the astigmatism test chart is rotated by 360 degrees, prior to a second phase in which a rotation angle of the astigmatism test chart is adjusted according to visual perception of the examinee of the astigmatism test chart.
(9) The ophthalmologic apparatus according to any one of above (1) to (8), further including an examinee's controller including a dial member that is rotated by the examinee, and wherein the control portion is configured to control the display portion according to a rotation angle of the dial member.
(10) A method for controlling an ophthalmologic apparatus,
   wherein the ophthalmologic apparatus includes:
   a display portion that is configured to display an astigmatism test chart for obtaining an astigmatism axis angle of an examinee, and
   a control portion that is configured to control the display portion to display and rotate the astigmatism test chart on the display portion,
   wherein the method includes displaying the astigmatism test chart on the display portion, the astigmatism test chart including a first visual target including a plurality of first straight lines that are arranged in parallel with each other at a predetermined interval, and a second visual target including a plurality of second straight lines that are arranged in parallel with each other at a predetermined interval and extend in a direction orthogonal to a direction of the first straight lines, and
   wherein the displaying the astigmatism test chart includes:
      displaying the first visual target in a circular first area, and
      displaying the second visual target in an annular second area surrounding the first area.
(11) The method according to above (10), wherein the ophthalmologic apparatus further includes an examinee's controller that is configured to receive an operation of the examinee,
   wherein the method further includes changing at least one of a width of the first straight lines, an interval between the first straight lines, a width of the second straight lines, and an interval between the second straight lines based on the operation of the examinee on the examinee's controller.
(12) The method according to above (10) or (11), wherein the ophthalmologic apparatus further includes a measurement optical system that is arranged between a subject eye of the examinee and the astigmatism test chart, the measurement optical system including an optical element whose power and axial direction are changeable,
   wherein the method further includes:
   displaying the astigmatism test chart on the display portion,
   rotating the astigmatism test chart displayed on the display portion,
   stopping rotation of the astigmatism test chart when the examinee clearly sees the first visual target,
   setting the axial direction of the optical element at a predetermined angle set based on an inclination direction of the first visual target when the rotation of the astigmatism test chart is stopped; and
   changing the power of the optical element so that the examinee clearly sees the second visual target when the axial direction of the optical element is set at the predetermined angle.

### List of Reference Signs

- 1, 1A: ophthalmologic apparatus
- 10: subjective optometric device (measurement optical system)
- 10L: optometric unit
- 10R: optometric unit
- 20: visual target presentation device
- 22: display portion
- 22a: visual target presentation area
- 30: operation device
- 31: first controller
- 32: examiner's operation portion
- 33: examiner's display portion
- 34, 80: control portion
- 35: second controller (examinee's controller)
- 36: examinee's operation portion
- 36a: dial member
- 36b: push button
- 37: second control portion
- 41: radial chart
- 42: radial line
- 43: marker
- 45, 45A: astigmatism test chart
- 46: first visual target
- 46a: first straight line
- 47: second visual target
- 47a: second straight line
- 48: first area
- 49: second area
- E: examiner
- S: examinee

## Claims

1. An ophthalmologic apparatus (1, 1A) comprising:
a display portion (22) that is configured to display an astigmatism test chart (45, 45A) for obtaining an astigmatism axis angle of an examinee (S); and
a control portion (34, 80) that is configured to control the display portion (22) to display and rotate the astigmatism test chart (45, 45A) on the display portion (22),
wherein the astigmatism test chart (45, 45A) comprises:
a first visual target (46) comprising a plurality of first straight lines (46a) that are arranged in parallel with each other at a predetermined interval, and
a second visual target (47) comprising a plurality of second straight lines (47a) that are arranged in parallel with each other at a predetermined interval and extend in a direction orthogonal to a direction of the first straight lines (46a),
wherein the first visual target (46) is displayed in a circular first area (48), and the second visual target (47) is displayed in an annular second area (49) surrounding the first area (48).

2. The ophthalmologic apparatus (1, 1A) according to claim 1, further comprising an examinee's controller (35) that is configured to receive an operation of the examinee (S),
wherein the control portion (34, 80) is configured to change at least one of a width of the first straight lines (46a), an interval between the first straight lines (46a), a width of the second straight lines (47a), and an interval between the second straight lines (47a) based on the operation of the examinee (S) on the examinee's controller (35).

3. The ophthalmologic apparatus (1, 1A) according to claim 1 or 2, wherein at least a part of an outline of the first and second straight lines (46a, 47a) of the astigmatism test chart (45, 45A) is blurred.

4. The ophthalmologic apparatus (1, 1A) according to claim 3, wherein brightness in a width direction of at least a part of the first and second straight lines (46a, 47a) in the astigmatism test chart (45, 45A) changes according to a sinusoidal function.

5. The ophthalmologic apparatus (1, 1A) according to any one of claims 1 to 4, further comprising a measurement optical system (10) arranged between a subject eye of the examinee (S) and the astigmatism test chart (45, 45A), the measurement optical system (10) comprising an optical element whose power and axial direction are changeable,
wherein the control portion (34, 80) is configured to control the display portion (22) to display and rotate the astigmatism test chart (45, 45A) on the display portion (22) and to stop rotation of the astigmatism test chart (45, 45A) when the examinee (S) clearly sees the first visual target (46), and
wherein the control portion (34, 80) is configured to control the measurement optical system (10) to set the axial direction of the optical element at a predetermined angle set based on an inclination direction of the first visual target (46) in a state where the rotation of the astigmatism test chart (45, 45A) is stopped, and to change the power of the optical element so that the examinee (S) clearly sees the second visual target (47).

6. The ophthalmologic apparatus (1, 1A) according to any one of claims 1 to 5, wherein the control portion (34, 80) is configured to control the display portion (22) to display a radial chart (41) comprising a plurality of radial lines (42) extending in a radial direction, and
wherein the control portion (34, 80) is configured to start rotation of the astigmatism test chart (45, 45A) from a position where the first straight lines (46a) are along the radial line (42) clearly seen by the examinee (S).

7. The ophthalmologic apparatus (1, 1A) according to claim 6, wherein the control portion (34, 80) is configured to control the display portion (22) to simultaneously display the radial chart (41) and a marker (43) in vicinity of a distal end of one of the radial lines (42) of the radial chart (41), and
wherein the control portion (34, 80) is configured to control the display portion (22) to sequentially move the marker (43) and then fix the marker (43) in vicinity of a distal end of the radial line (42) that is clearly seen by the examinee (S).

8. The ophthalmologic apparatus (1, 1A) according to any one of claims 1 to 7, wherein when the display portion (22) displays and rotates the astigmatism test chart (45, 45A), the control portion (34, 80) is configured to perform a first phase in which the astigmatism test chart (45, 45A) is rotated by 360 degrees, prior to a second phase in which a rotation angle of the astigmatism test chart (45, 45A) is adjusted according to visual perception of the examinee (S) of the astigmatism test chart (45, 45A).

9. The ophthalmologic apparatus (1, 1A) according to any one of claims 1 to 8, further comprising an examinee's controller (35) comprising a dial member (36a) that is rotated by the examinee (S), and
wherein the control portion (34, 80) is configured to control the display portion (22) according to a rotation angle of the dial member (36a).

10. A method for controlling an ophthalmologic apparatus (1, 1A),
wherein the ophthalmologic apparatus (1, 1A) comprises:
a display portion (22) that is configured to display an astigmatism test chart (45, 45A) for obtaining an astigmatism axis angle of an examinee (S); and
a control portion (34, 80) that is configured to control the display portion (22) to display and rotate the astigmatism test chart (45, 45A) on the display portion (22),
wherein the method comprises displaying the astigmatism test chart (45, 45A) on the display portion (22), the astigmatism test chart (45, 45A) comprising a first visual target (46) comprising a plurality of first straight lines (46a) that are arranged in parallel with each other at a predetermined interval, and a second visual target (47) comprising a plurality of second straight lines (47a) that are arranged in parallel with each other at a predetermined interval and extend in a direction orthogonal to a direction of the first straight lines (46a), and
wherein the displaying the astigmatism test chart (45, 45A) comprises:
displaying the first visual target (46) in a circular first area (48), and
displaying the second visual target (47) in an annular second area (49) surrounding the first area (48).

11. The method according to claim 10, wherein the ophthalmologic apparatus (1, 1A) further comprises an examinee's controller (35) that is configured to receive an operation of the examinee (S),
wherein the method further comprises changing at least one of a width of the first straight lines (46a), an interval between the first straight lines (46a), a width of the second straight lines (47a), and an interval between the second straight lines (47a) based on the operation of the examinee (S) on the examinee's controller (35).

12. The method according to claim 10 or 11, wherein the ophthalmologic apparatus (1, 1A) further comprises a measurement optical system (10) that is arranged between a subject eye of the examinee (S) and the astigmatism test chart (45, 45A), the measurement optical system (10) comprising an optical element whose power and axial direction are changeable,
wherein the method further comprises:
displaying the astigmatism test chart (45, 45A) on the display portion (22);
rotating the astigmatism test chart (45, 45A) displayed on the display portion (22);
stopping rotation of the astigmatism test chart (45, 45A) when the examinee (S) clearly sees the first visual target (46);
setting the axial direction of the optical element at a predetermined angle set based on an inclination direction of the first visual target (46) when the rotation of the astigmatism test chart (45, 45A) is stopped; and
changing the power of the optical element so that the examinee (S) clearly sees the second visual target (47) when the axial direction of the optical element is set at the predetermined angle.
